# FASCICULE DE BREVET EUROPEEN

(11) **EP 2 872 221 B1**
(45) Date de publication et mention de la délivrance du brevet: **29.08.2018**
(21) Numéro de dépôt: 13765473.7
(22) Date de dépôt: 09.07.2013
(51) Int. Cl.: A61Q 19/08, A61K 8/97

(54) **UTILISATION COSMETIQUE D'UN EXTRAIT D'HARUNGANA MADAGASCARIENSIS**
KOSMETISCHE VERWENDUNG EINES EXTRAKTES AUS HARUNGANA MADAGASCARIENSIS
COSMETIC USE FOR A HARUNGANA MADAGASCARIENSIS EXTRACT

(30) Priorité: 10.07.2012 FR 1201953
(43) Date de publication de la demande: 20.05.2015
(73) Titulaire: Laboratoires Clarins, 92200 Neuilly sur Seine (FR)
(72) Inventeur: COURTIN, Olivier, F-92200 Neuilly Sur Seine (FR)
(74) Mandataire: Gevers & Orès
(86) Numéro de dépôt international: PCT/IB2013/055623
(87) Numéro de publication internationale: WO 2014/009874

(56) Documents cités:
- EP-A2- 0 985 416
- WO-A2-2004/075867
- OKOLI A S ET AL: "Antibacterial activity of Harungana madagascariensis leaf extracts.", PHYTOTHERAPY RESEARCH : PTR MAR 2002, vol. 16, no. 2, mars 2002 (2002-03), pages 174-179, XP002698502, ISSN: 0951-418X
- AGBOR GABRIEL A ET AL: "Medicinal plants can be good source of antioxidants: case study in Cameroon.", PAKISTAN JOURNAL OF BIOLOGICAL SCIENCES: PJBS 15 FEB 2007, vol. 10, no. 4, 15 février 2007 (2007-02-15), pages 537-544, XP002698503, ISSN: 1028-8880
- KISANGAU D P ET AL: "In vitro antimicrobial assay of plants used in traditional medicine in Bukoba Rural district, Tanzania.", AFRICAN JOURNAL OF TRADITIONAL, COMPLEMENTARY, AND ALTERNATIVE MEDICINES : AJTCAM / AFRICAN NETWORKS ON ETHNOMEDICINES 2007, vol. 4, no. 4, 2007, pages 510-523, XP002698504, ISSN: 0189-6016
- BIAPA PROSPER-CABRAL N ET AL: "Phytochemical studies and antioxidant properties of four medicinal plants used in Cameroon.", AFRICAN JOURNAL OF TRADITIONAL, COMPLEMENTARY, AND ALTERNATIVE MEDICINES : AJTCAM / AFRICAN NETWORKS ON ETHNOMEDICINES 2007, vol. 4, no. 4, 2007, pages 495-500, XP002698505, ISSN: 0189-6016
- MOULARI B ET AL: "Isolation and in vitro antibacterial activity of astilbin, the bioactive flavanone from the leaves of Harungana madagascariensis Lam. ex Poir. (Hypericaceae).", JOURNAL OF ETHNOPHARMACOLOGY 30 JUN 2006, vol. 106, no. 2, 30 juin 2006 (2006-06-30) , pages 272-278, XP027939647, ISSN: 0378-8741

## Description

La présente invention concerne l'utilisation cosmétique d'un extrait de feuilles d'Harungana madagascariensis ou d'une composition comprenant ledit extrait, en particulier pour retarder ou pour lutter contre le vieillissement de la peau et/ou l'apparition des signes de vieillissement de la peau, tel que décrit dans les revendications. En effet, l'utilisation de cet extrait ou de cette composition permet de favoriser la synthèse de collagène ; ainsi que de protéger les protéines cutanées de la glycation.

La peau est un organe particulier du corps humain. De fine épaisseur, elle est très étendue puisqu'elle recouvre toute la surface du corps et totalise chez l'adulte une surface d'environ 1,6 m². Elle a pour fonction de protéger les tissus profonds du milieu extérieur, tant au niveau de la pénétration physique de corps étrangers, que de l'immunité, de la régulation de la température, et de la déperdition de fluides. Enfin, les contraintes mécaniques auxquelles elle est soumise en permanence lui imposent, plus que tout autre organe, une structure solide et cohérente associée à une grande souplesse. Malgré quelques différences d'aspect suivant le site anatomique, elle présente toujours la même structure morphologique de base. Elle est constituée à sa surface de l'épiderme, en profondeur du derme, et de la jonction dermo-épidermique qui les sépare et les relie.

Le rôle du derme est de maintenir la souplesse et la résistance de la peau. C'est un tissu conjonctif dense, fibreux et élastique reposant sur une couche graisseuse appelée hypoderme, et au sein duquel baignent des cellules mésenchymateuses, les fibroblastes. Ceux-ci sont responsables de la synthèse des constituants de la matrice extracellulaire qui les entoure, véritable tissu de soutien de la peau. La matrice extracellulaire est structurée par une trame fibreuse faite de faisceau de collagène et de fibres élastiques (élastine et fibrilline), en vagues parallèles à la surface de la peau.

Au-delà de son rôle physique de soutien de l'épiderme, le derme assure d'autres fonctions essentielles. Sa vascularisation importante lui permet de réaliser des échanges nutritifs avec l'épiderme. Elle lui permet aussi d'être riche en cellules immunitaires de type macrophages et cellules dendritiques, acteurs essentiels de la défense immunitaire de la peau. De plus, c'est un tissu innervé possédant des récepteurs sensitifs responsables du sens du toucher. Enfin, il intervient dans les phénomènes de cicatrisation, et de régulation de la prolifération et de la différenciation de kératinocytes en synthétisant des cytokines et des facteurs de croissance solubles à destination de l'épiderme.

Le collagène confère à la peau sa résistance aux tensions et à la traction. L'élastine permet à la peau de s'étirer et de revenir en place après déformation. Elle confère son élasticité au revêtement cutané. La matrice extracellulaire contient aussi des protéoglycanes et des glycosaminoglycanes, qui forment une substance fondamentale très hydratée, dans laquelle sont noyées les protéines fibreuses. Enfin, des glycoprotéines de structure assurent l'interface entre la matrice et les cellules en liant les intégrines membranaires.

La matrice extracellulaire de la peau est la matière interstitielle qui entoure les structures cutanées et soutient l'épiderme. Au sein du réseau des protéines fibreuses, le collagène est le principal composant insoluble qui contribue aux propriétés mécaniques de la peau. Les « fibres de collagène » du derme sont constituées respectivement de collagène I et de collagène III, autour d'un axe composé de collagène V. Ces collagènes appartiennent au groupe des collagènes fibrillaires. Chez l'adulte, le collagène I est, en moyenne, six fois plus abondant que le collagène III.

Les fibrilles de collagène I ont une énorme résistance à la traction, et peuvent donc être étirées sans être cassées. Ces fibrilles se disposent côte à côte en rangées parallèles, appelées fibres de collagène. Le collagène III se compose de trois chaînes alpha 1(III) et est prédominant dans la peau foetale et les structures extensibles. La proportion de collagène I augmente quand on se rapproche de l'hypoderme. La matrice extracellulaire contient également de la fibronectine, de la laminine, de l'élastine ainsi que de la substance fondamentale de protéoglycanes.

Malgré la grande stabilité de ses constituants, la matrice extracellulaire est une structure dynamique produite en majeure partie localement par les cellules du tissu conjonctif. Dans ce contexte, les fibroblastes jouent un rôle essentiel dans l'entretien et la régénération de ce tissu. Leur capacité à synthétiser et à dégrader le collagène et d'autres macromolécules de la matrice extracellulaire, ou encore à organiser ou à orienter les fibres est critique dans l'homéostasie du tissu cutané.

Pendant le vieillissement, certaines anomalies de la communication entre les cellules et la matrice environnante peuvent conduire à une pléthore de modifications qualitatives et quantitatives de la structure de la matrice extracellulaire. Par exemple, Le rapport collagène I sur collagène III diminue au cours du vieillissement. On assiste aussi à une augmentation exponentielle de pontages chimiques entre les fibres de collagène dus à la réaction de glycation non enzymatique de Maillard.

Ce pontage chimique du collagène âgé aboutit à une rigidification croissante de fibres qui les rend plus résistantes à l'attaque par les collagénases et par les radicaux libres. Sa dégradation et son renouvellement sont ainsi ralenties.

La glycation dépend des possibilités de rencontre entre les molécules de glucose circulantes et les groupements aminés libres existants soit à l'extrémité N-terminale des chaînes polypeptidiques, soit sur les chaînes latérales de lysine. Le mécanisme biochimique de cette réaction est bien connu et comporte deux phases : une phase précoce (initiation) qui est la réaction de sucres réducteurs (glucose, fructose) avec les fonctions amines terminales ou latérales des protéines constitutives tissulaires pour produire des composés appelés « Bases de Schiff ». Ces composés sont stabilisés ensuite par réarrangement d'Amadori en cétoamine. Elle est suivie d'une phase tardive (propagation et terminaison) où les fonctions cétoamine sont oxydées en désoxyosone en présence d'oxygène et réagissent avec d'autres acides aminés basiques tels que l'arginine ou la lysine appartenant à d'autres protéines (albumine, lipoprotéines, immunoglobuline). Le résultat est la formation de complexes avec des pontages finaux du type cycles pentosidine ou furoyl 6.3. furonyl 1.4 imidazole.

La réaction de glycation protéique constitue un des processus majeur de vieillissement cutané induit. Elle aboutit en effet à la formation de liaisons croisées irréversibles entre les protéines qui vont s'accumuler avec l'âge ou dans des états pathologiques (diabète, stress oxydatif...) .

Les protéines glyquées ainsi pontées perdent leur fonctionnalité biologique ; les tissus se rigidifient et se sclérosent.

La demanderesse a mis en évidence de manière surprenante qu'un extrait d'Harungana madagascariensis ou qu'une composition comprenant un tel extrait protège du vieillissement cutané en favorisant la synthèse de collagène et en protégeant les protéines cutanées de la glycation. On peut donc utiliser un extrait d'Harungana madagascariensis comme agent actif anti vieillissement cutané, comme agent actif favorisant la synthèse de collagène et/ou comme agent anti glycation.

Harungana madagascariensis est un arbre de la famille des hypericaceae largement présent à Madagascar.

Cette plante est utilisée traditionnellement comme colorant jaune et à des fins thérapeutiques notamment comme antibactérien et anti-inflammatoire (voir par exemple Okoli, S. et al. (2002) "Antibacterial activity of Harungana madagascariensis leaf extracts", Phytotherapy Research, vol. 16, no. 2, pp. 174-179; Moulari, B. et al. (2006) "Isolation and in vitro antibacterial activity of astilbin, the bioactive flavanone from the leaves of Harungana madagascariensis Lam. ex Poir. (Hypericaceae)", vol. 106, no. 2, pp. 2727-278). L'utilisation selon la présente invention met préférentiellement en oeuvre un extrait d'Harungana madagascariensis ou une composition cosmétique, comprenant un tel extrait.

L'extrait utilisé est un extrait de feuilles d'Harungana madagascariensis. De façon avantageuse, cet extrait est un extrait aqueux et de préférence il est stabilisé par de la glycérine. De préférence il s'agit d'un extrait aqueux obtenu selon un procédé comprenant au moins les étapes suivantes :
- séchage des feuilles ;
- extraction à l'eau des feuilles ;
- maturation puis filtration ;
- évaporation du solvant d'extraction;
- ajustement de la concentration (ajout de glycérine).

L'extrait d'Harungana madagascariensis utilisé selon l'invention, est un liquide visqueux marron-rouge et d'odeur caractéristique. Il présente les caractéristiques analytiques suivantes :
pH (à 1% dans l'eau): 4 - 6
Densité à 20 °C : 1,2 - 1,4
Indice de réfraction : 1,4 -1,6
Rapport plante/extrait = 10/4

De préférence la composition cosmétique utilisée selon l'invention comprend de 0,01 à 10% d'extrait d'Harungana madagascariensis en poids de matière sèche par rapport au poids total de la composition. Avantageusement, la composition comprend de 0,01 à 5% d'extrait d'Harungana madagascariensis en poids de matière sèche par rapport au poids total de la composition.

Les compositions utilisées selon l'invention peuvent encore comprendre un ou plusieurs agents de formulation ou additifs d'usage connu et classique dans les compositions cosmétiques et dermatologiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc... Grâce à ses connaissances en matière de cosmétique, l'homme du métier saura quels agents de formulation ajouter aux compositions de l'invention et en quelles quantités en fonction des propriétés recherchées.

Les compositions utilisées selon l'invention peuvent se présenter sous toute forme connue de l'homme du métier dans le domaine de la cosmétologie et de la dermatologie sans autre restriction galénique que l'application sur le visage et sur le corps. De façon avantageuse, les compositions selon l'invention se présentent sous la forme d'un gel, d'une crème, d'une lotion, d'une huile, d'un lait, d'un spray, etc...

La demanderesse a démontré que l'extrait d'Harungana madagascariensis utilisé selon l'invention ainsi que la composition cosmétique le comprenant peuvent être utilisés pour favoriser la synthèse de collagène ; ou encore pour protéger les protéines cutanées de la glycation.

En conséquence l'extrait d'Harungana madagascariensis ou la composition cosmétique le comprenant selon l'invention peuvent être utilisés pour prévenir, pour retarder, pour lutter contre, pour traiter ou réduire le vieillissement de la peau et/ou l'apparition des signes de vieillissement de la peau, tel que décrit dans les revendications. On entend par signes de vieillissement de la peau : les rides, les ridules, le relâchement cutané, la perte d'élasticité des fibres cutanées, une peau flétrie, une peau amincie, et une peau terne et/ou sans éclat.

Grace son action sur l'induction de la synthèse du collagène dans la peau, selon une variante préférée de l'invention, l'extrait d'Harungana madagascariensis peut être utilisé pour réparer les manifestations cutanées du vieillissement ; en particulier estomper les rides et les ridules, ou encore re-densifier une peau flétrie ou amincie.

L'extrait d'Harungana madagascariensis utilisé selon l'invention et la composition cosmétique le comprenant peuvent également être utilisés pour redensifier et/ou raffermir et/ou tonifier la peau.

L'extrait d'Harungana madagascariensis selon l'invention et la composition cosmétique le comprenant sont utilisés par application sur la peau ; il peut s'agir indifféremment de la peau du visage ou du corps.

Lorsque qu'il s'agit de la peau du visage, c'est préférentiellement la peau de la face, du cou, du décolleté et/ou du contour des yeux.

Selon une variante particulière, L'extrait d'Harungana madagascariensis selon l'invention et la composition cosmétique le comprenant sont appliqués sur la peau du corps pour la raffermir et/ou la tonifier, par exemple dans le cadre d'un amincissement.

Les exemples ci-après concernent, d'une part l'évaluation de l'effet d'un extrait d'Harungana madagascariensis sur la synthèse de collagène I, et sur son effet anti glycation des protéines et d'autre part des compositions utilisables selon la présente invention.

Les exemples font référence aux figures suivantes dans lesquelles :
- La figure 1 représente l'immunofluorescence du collagène I (TRITC) pour le témoin (panneau A), le témoin positif le rétinol à 10⁻⁵ M (panneau B) et pour différentes concentrations d'extrait d'Harungana madagascariensis 0,001% (panneau C), 0,005% (panneau D) et 0,01% (panneau E) (Observations en microscopie optique à fluorescence grossissement x200).

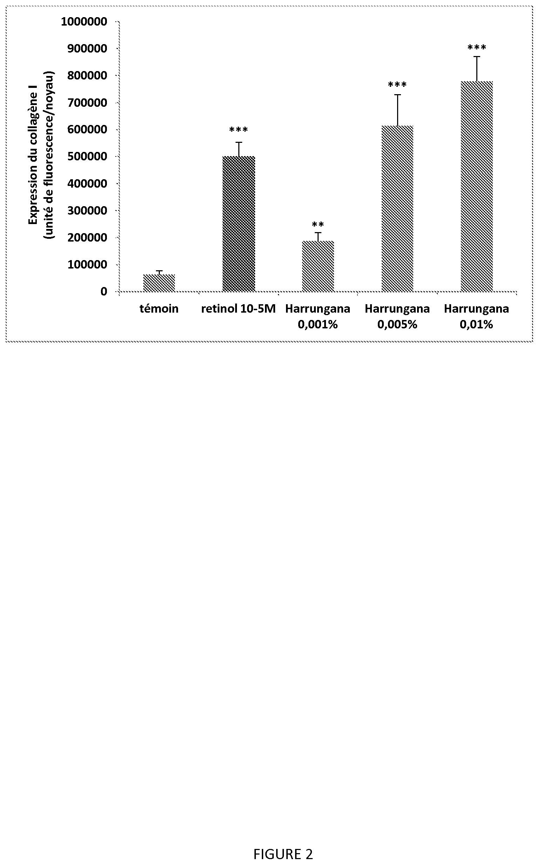

- La figure 2 représente l'expression du collagène I exprimé en unité de fluorescence par noyau.

### I.EVALUATION DE L'ACTIVITE D'UN EXTRAIT D'HARUNGANA MADAGASCARIENSIS SUR LA SECRETION DU COLLAGENE DE TYPE I

### A.MATERIEL ET METHODE

Les fibroblastes sont ensemencés dans des plaques 6 puits avec lamelle de verre à raison de 70.000 cellules par boîte dans du milieu 'Minimum Essential Médium' (MEM). 48h après, les cellules sont traitées ou non (témoin) par l'extrait d'Harungana madagascariensis à 0,001%, 0,005% et 0,01% (on utilise comme témoin positif le rétinol à la concentration 10⁻⁵ M) et incubées à l'étuve pendant 72 heures à 37°C, 5%CO₂.

Chaque tapis cellulaire est ensuite rincé, fixé au méthanol (-20°C) avant de procéder à la révélation du collagène de type I.

Les cellules sont incubées avec le premier anticorps dirigé contre la protéine d'intérêt puis avec l'anticorps secondaire dirigé contre le 1^{er} anticorps couplé au fluorochrome TRITC (Tetra methyl Rhodamine Iso Thio Cyanate, dérivé de la Rhodamine). Le temps d'incubation entre chaque anticorps est d'une heure. Les noyaux cellulaires sont révélés par un marquage au Di Aminido Phenyl lndol (Dapi).

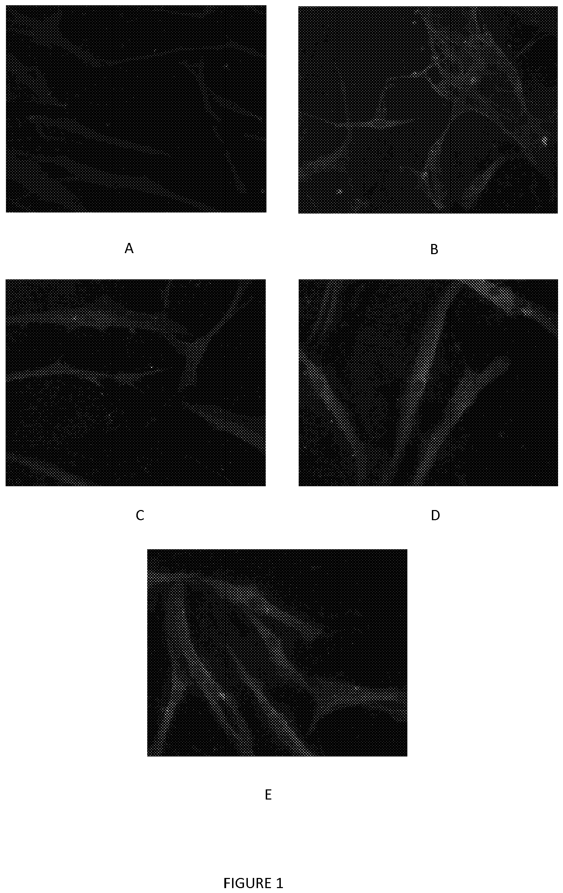

Les lamelles sont montées sur lame de verre puis observées au microscope à fluorescence (Nikon Eclipse 50i). Plusieurs champs de la population cellulaire sont pris en photo. Les images de fluorescence TRITC indiquent les régions de localisation de la protéine d'intérêt. Les images de fluorescence DAPI indiquent les noyaux cellulaires et donc le nombre de cellules par champ. Les clichés photographiques, tels que ceux représentés à la figure 1, sont analysés par le logiciel d'analyse d'image NIS Elements (Nikon).

### B.RESULTATS

Cette étude démontre que l'extrait d'Harungana madagascariensis favorise de manière significative la synthèse du collagène de type I par les fibroblastes de manière dose-dépendante. L'extrait d'Harungana madagascariensis favorise la synthèse du composant majoritaire de la matrice extracellulaire, le collagène de type I. Ces propriétés confèrent donc à cet extrait un rôle anti-âge.

### II.EVALUATION DE LA PROTECTION D'UN EXTRAIT D'HARUNGANA MADAGASCARIENSIS VIS A VIS DE LA REACTION DE LA GLYCATION PROTEIQUE

Un modèle *in vitro* a été retenu pour évaluer les effets de l'extrait d'Harungana madagascariensis. Il repose sur la mesure de la formation des dérivés de la réaction de glycation entre les groupements aminés libres de l'albumine bovine et le glucose. Certains de ces dérivés, les produits terminaux de glycation avancés, sont fluorescents. La réaction est mesurée après 8 jours d'incubation. Une molécule de référence, l'aminoguanidine, est testée en parallèle.

### A.MATERIEL ET METHODE

Le système d'essai est le mélange réactionnel contenant de l'albumine bovine (0,5 g/ml) et du glucose (500 mM) dans un tampon phosphate (0,2 M, pH = 7,4).

Les produits sont mélangés au système d'essai dans des tubes stériles ; ces derniers sont recouverts de papier aluminium (la réaction doit se faire à l'abri de la lumière). Pour les conditions expérimentales où certains réactifs ou produits ne seront pas présents, il faut compléter le volume du tube QSP avec de l'eau MilliQ.

Après avoir fermé les tubes avec du parafilm (la réaction doit se dérouler à l'abri de l'oxygène), les différents mélanges réactionnels sont placés dans une étuve sèche à 37°C pendant 8 jours.

Après les 8 jours d'incubation, 100 µl de chaque tube est prélevé et transféré dans une plaque noire 96 puits. La fluorescence est alors lue au FLUOstar (*BMG*) (excitation à 355 nm, émission à 460 nm).

### B.RESULTATS

Les résultats sont présentés dans le tableau qui suit ; ils sont exprimés en unité arbitraire de fluorescence. La fluorescence relative aux échantillons « produit + albumine bovine » (= interférence) sera soustraite des données « produit + albumine bovine + Glucose ».

Ce tableau représente la formation de liaisons croisées de glycation exprimée en unité arbitraire de fluorescence (S.E = système d'essai, mélange réactionnel contenant de l'albumine bovine (0,5 g/ml) et du glucose (500 mM) dans un tampon phosphate (0,2 M, pH = 7,4)).

| | **Unité arbitraire de fluorescence** | **Protection (%)** |
|---|---|---|
| S.E. | 37673 | / |
| S.E.+ aminoguanidine 10 mM | 0 | 100 |
| S.E.+Harungana Madagascariensis 0,001% | 24737 | 34 |
| S.E.+Harungana Madagascariensis 0,005% | 28132 | 25 |
| S.E.+Harungana Madagascariensis 0,01% | 14894 | 60 |

Après 8 jours d'incubation d'albumine bovine avec une surcharge en glucose, la formation de liaisons croisées de glycation est observée. Elle a été évaluée en tirant parti des propriétés de fluorescence de l'adduit albumine bovine-glucose.

L'extrait d'Harungana madagascariensis présent dans le système d'essai empêche la liaison croisée entre l'albumine bovine et le glucose de manière non négligeable.

### III.EXEMPLES DE FORMULATION

Les pourcentages sont des pourcentages en poids par rapport au poids total de la composition.

### GEL NETTOYANT VISAGE

| | **%** |
|---|---|
| HECTORITE | 1,50 |
| GLYCOLS | 2,00 |
| XANTHAN GUM | 0,25 |
| SODIUM LAURETH SULFATE | 3,00 |
| SODIUM STEAROYL & SODIUM COCOYL GLUTAMATE | 7,00 |
| BEURRE DE KARITE | 0,50 |
| SODIUM COCOAMPHO ACETATE | 10,00 |
| GLYCOL DISTEARATE | 4,00 |
| LANETTE SX | 9,00 |
| HARUNGANA MADAGASCARENSIS | 0,50 |
| CONSERVATEURS | Q.S. |
| DISODIUM EDTA | 0,10 |
| PARFUM | Q.S. |
| EAU DEMINERALISEE | Q.S.P 100 |

### LOTION TONIQUE

| | **%** |
|---|---|
| XANTHAN GUM | 0,15 |
| CARBOMER | 0,15 |
| BIOSACCHARIDE GUM-1 | 10,00 |
| LUBRAJEL | 10,00 |
| HARUNGANA MADAGASCARENSIS | 1,00 |
| PEG-40 HYDROGENATED CASTOR OIL | 0,40 |
| CONSERVATEURS | Q.S. |
| PARFUM | Q.S. |
| EAU DEMINERALISEE | Q.S.P 100 |

### CRÈME DE NUIT ANTI-ÂGE

| | **%** |
|---|---|
| CETEARYL ALCOHOL & CETEARYL GLUCOSIDE | 4,50 |
| GLYCERYL STEARATE & PEG-100 STEARATE | 2,00 |
| STEARETH-21 | 0,50 |
| HYDROGENATED COCO-GLYCERIDES | 2,00 |
| CAPRYLIC/CAPRIC TRIGLYCERIDES | 8,00 |
| BEURRE DE KARITE | 2,00 |
| DIMETHICONE | 0,50 |
| CARBOMER | 0,30 |
| GLYCOLS | 5,00 |
| SEPIGEL 305 | 0,30 |
| HARUNGANA MADAGASCARENSIS | 3,00 |
| SODIUM HYDROXIDE | 0,08 |
| CONSERVATEURS | Q.S. |
| PARFUM | Q.S. |
| EAU DEMINERALISEE | Q.S.P 100 |

### SERUM ANTI-ÂGE

| | **%** |
|---|---|
| COVACRYL MV 60 | 0,70 |
| FUCOGEL | 8,00 |
| HARUNGANA MADAGASCARENSIS | 5,00 |
| GLYCOLS | 10,00 |
| CONSERVATEURS | Q.S. |
| PARFUM | Q.S. |
| EAU DEMINERALISEE | Q.S.P 100 |

## Revendications

1. Utilisation cosmétique d'une composition cosmétique comprenant un extrait de feuilles d'Harungana madagascariensis ou d'un extrait de feuilles d'Harungana madagascariensis comme agent favorisant la synthèse de collagène et/ou protégeant les protéines cutanées de la glycation pour retarder, lutter contre, traiter ou réduire l'apparition des signes de vieillissement de la peau sélectionnés parmi les rides, les ridules, le relâchement cutané, la perte d'élasticité des fibres cutanées, une peau flétrie, une peau amincie, et/ou estomper les rides et les ridules et/ou re-densifier une peau flétrie ou amincie et/ou redensifier et/ou raffermir et/ou tonifier la peau du visage et/ou du corps.

2. Utilisation cosmétique selon l'une quelconque des revendications précédentes, dans laquelle l'extrait d'Harungana madagascariensis est un extrait aqueux.

3. Utilisation cosmétique selon la revendication 2, dans laquelle l'extrait d'Harungana madagascariensis est stabilisé par de la glycérine.

4. Utilisation cosmétique selon l'une quelconque des revendications précédentes, **caractérisée en ce que** ledit extrait est formulé dans une composition cosmétique qui comprend de 0,01 à 10% dudit extrait en poids de matière sèche par rapport au poids total de la composition, de préférence de 0,01 à 5% dudit extrait en poids de matière sèche par rapport au poids total de la composition.

5. Utilisation cosmétique selon la revendication 4, dans laquelle ladite composition cosmétique comprend en outre un ou plusieurs agents de formulation ou additifs tels que des adoucissants, des colorants, des agents filmogènes, des tensio-actifs, des parfums, des conservateurs, des émulsionnants, des huiles, des glycols, filtres UV, et des vitamines.

## Patentansprüche

1. Kosmetische Verwendung einer kosmetischen Zusammensetzung, umfassend einen Extrakt von Blättern aus *Harungana madagascariensis* oder einen Extrakt von Blättern aus *Harungana madagascariensis* als Mittel, das die Synthese von Collagen begünstigt und/oder die Hautproteine der Glykierung schützt, um das Auftreten von Zeichen der Alterung der Haut zu verzögern, dagegen anzukämpfen, diese zu behandeln oder zu reduzieren, ausgewählt aus den Falten, den Fältchen, der Hauterschlaffung, dem Verlust der Elastizität der Hautfasern, welker Haut, einer verdünnten Haut, und/oder die Falten und die Fältchen zu mildern und/oder eine welke oder verdünnte Haut wieder zu verdichten und /oder die Haut des Gesichts und/oder des Körpers wieder zu verdichten und/oder wieder zu straffen und/oder zu kräftigen.

2. Kosmetische Verwendung nach einem der vorhergehenden Ansprüche, wobei der Extrakt aus *Harungana madagascariensis* ein wässriger Extrakt ist.

3. Kosmetische Verwendung nach Anspruch 2, wobei der Extrakt aus *Harungana madagascariensis* durch Glyzerin stabilisiert ist.

4. Kosmetische Verwendung nach einem der vorhergehenden Ansprüche, **dadurch gekennzeichnet, dass** der Extrakt in einer kosmetischen Zusammensetzung formuliert ist, die von 0,01 bis 10 % des Extrakts in Gewicht der Trockenmasse mit Bezug auf das Gesamtgewicht der Zusammensetzung umfasst, vorzugsweise von 0,01 bis 5 % des Extrakts in Gewicht der Trockenmasse mit Bezug auf das Gesamtgewicht der Zusammensetzung.

5. Kosmetische Verwendung nach Anspruch 4, wobei die kosmetische Zusammensetzung außerdem ein oder mehrere Formulierungshilfsmittel oder Zusatzstoffe wie z. B. Weichmacher, Farbstoffe, filmbildende Mittel, Tenside, Parfüms, Konservierungsstoffe, Emulsionsmittel, Öle, Glykole, UV-Filter und Vitamine umfasst.

## Claims

1. Cosmetic use of a cosmetic composition comprising a Harungana madagascariensis leaf extract or a Harungana madagascariensis extract as an agent promoting collagen synthesis and/or protecting skin proteins from glycation in order to delay, combat, treat or reduce the appearance of the signs of skin ageing selected from wrinkles, fine lines, slackening of the skin, loss of skin fibre elasticity, loose skin, thinned skin, and/or diminish the visible appearance of wrinkles and fine lines and/or loose or thinned skin and/or replenish and/or firm and/or tone the skin of the face and/or body.

2. Cosmetic use according to any one of the preceding claims, wherein the Harungana madagascariensis extract is an aqueous extract.

3. Cosmetic use according to claim 2, wherein the Harungana madagascariensis extract is stabilised with glycerine.

4. Cosmetic use according to any one of the preceding claims, **characterised in that** said extract is formulated in a cosmetic composition comprising from 0.01 to 10% of said extract by weight of dry matter with respect to the total weight of the composition, preferably from 0.01 to 5% of said extract by weight of dry matter with respect to the total weight of the composition.

5. Cosmetic use according to claim 4, wherein said cosmetic composition further comprises one or a plurality of formulation agents or additives such as softeners, colorants, film-forming agents, surfactants, fragrances, preservatives, emulsifiers, oils, glycols, UV filters, and vitamins.
